# EUROPEAN PATENT APPLICATION

(11) **EP 0 965 969 A1**
(43) Date of publication of application: **22.12.1999**
(21) Application number: 98810560.7
(22) Date of filing: 18.06.1998
(51) Int. Cl.: G09B 21/00, A61F 11/04, A61F 9/08

(54) **The method of conveying information to the nervous system of a person**

(71) Applicant: Fingerov, Gavriil Mikhailovich, Moscow, 113055 (RU)
(72) Inventor: Fingerov, Gavriil Mikhailovich, 113055 Moscow (RU); Ilushenko, Sergey Vladimirovich, 117296 Moscow (RU)
(74) Representative: AMMANN PATENTANWÄLTE AG BERN AMMANN INGENIEURS-CONSEILS EN PROPRIETE INTELLECTUELLE SA BERNE AMMANN PATENT ATTORNEYS LTD BERNE

(57) **Abstract**

The method includes disintegration of the initial information into separate streams, coding each stream as a sequence of signals and transmitting the signals of each stream to the separate fixed areas situated on the integument of a person forming in total skin sensitive information zone, the function of coding being cyclically changed in the process of conveying information, i.e. conveying the visual information is carried out with electrical signals discretely frame by frame with cyclical frame by frame changing the dependence of current intensity and/or voltage of signals from the brightness of corresponding fragments of the frames ensuring integration of perceiving frames within the limits of a cycle with choosing time characteristics of the cycles; the dependence of current intensity and/or voltage of the signals from the brightness of the corresponding fragment is direct in a part of the cycle (positive frames) and is inverse in the other part of the cycle (negative frames), the number of the positive cycles can be more or less than that of the negative ones, the frequency of the signals' current in the negative and positive fragments of the cycle may be different.

## Description

### FIELD OF THE INVENTION

The invention relates to the methods of conveying information to the nervous system of a person. The invention can be used by people with the organs of sense affection, e. g. blind or deaf people.

### DESCRIPTION OF THE PRIOR ART

There exists a well-known method of conveying information to the nervous system of a person including disintegration of the initial information into separate streams, coding each stream as a sequence of signals and transmitting each stream signals to the separate fixed points situated on the integument of a person forming in total tactile information zone (see Nº WO 96/33481, 24 October, 1996).

The disadvantage of this well-known method is that during sending signals to the skin sensitive information zone the receptors adapt to the coming influences which results in reducing their sensitivity. Another disadvantage of the method is that strong signals not only activate the receptors of the areas of skin sensitive zone where they are being transmitted to but also the receptors of the adjacent points.

As a result the perception of relatively weak signals is suppressed by strong ones. So this method application is accompanied with reducing discernibility of the signals perception and the loss of information being sent to the skin sensitive zone.

### SUMMARY OF THE INVENTION

The purpose of the given invention is elimination of the above mentioned disadvantages. It can be achieved by the method of conveying information to the nervous system of a person including disintegration of the initial information into separate streams, coding every stream as a sequence of signals and transmitting the signals of each stream to separate fixed areas of tactile sensitivity of a person forming in total information zone, the function of coding being changed cyclically during the process of conveying information ensuring integration of information perception within the limits of a cycle by choosing the parameters of the function and the law of changing the function in time. E. g. conveying visual information is performed with electrical signals discretely frame by frame with cyclical frame by frame changing current intensity and for voltage and/or frequency of these parameters of the signals from the illumination of the corresponding frames fragments and integration of frames perception within the limits of a cycle is ensured with choosing time characteristics of the cycles; the dependence of current intensity and/or voltage and/or frequency of these parameters of the signals from the illumination of the core-spending fragment is direct (positive frames) for some fragments and is inverse (negative frames) for the other fragments and the number of the positive frames can be more or less than the number of the negative ones and the current frequencies in the positive and negative frames can differ.

Such realisation of the method suppresses the receptors' adaptation and permits to mark out the areas of weak signals separately.

Audial information conveyance can be carried out with vibrational signals, the pitch of sounds being coded with vibration frequency and the sound volume being coded with an amplitude with cycling changing of dependence of vibrostimulation amplitude from the corresponding sound frequencies amplitude; the dependence is direct (positive) in a part of acycle and is inverse (negative) in the other part and the integration of the cycle perception is ensured with choosing time characteristics of the cycle which permits to mark out relatively weak vibro-signals from the strong ones separately.

### DESCRIPTION OF THE PREFERRED EMBODIMENTS OF THE INVENTION

The versions of embodiments of the above-mentioned method, of the method related to transmitting the signals from the visual image to the skin sensitive information zone are given below. According to the given version of the method the image perceived by a TVcamera is being divided into fragments and each fragment is placed in correspondence with an area of the skin sensitive information zone. The information about the illumination of each fragment is to be coded as an electrical signal that is transmitted to the area of the skin sensitive zone corresponding to the given fragment. The function of coding information about the illumination of each fragment lies in establishing one- valued correspondence between the illumination of the fragment and the intensity of the signal corresponding to it. The intensity of the signal is to be determined in its turn with the strength of current impulse and/or voltage applied to the area of the skin sensitive information zone corresponding to the given fragment In such embodiment of the method the difference between the illumination of the fragments is coded with the difference of the intensity of electrical skin stimulation or electrical tactile stimulation of the information zone areas. The image signals are to be transmitted to the skin sensitive information zone with frames. The function of coding is to be changed cyclically to suppress the receptors' adaptation to the signals transmitted to them and for better perception of the information transmitted to the skin sensitive information zone. For example, a cycle of changing the function of coding may consist of 5 frames; in this case the function of coding is linear for the first frame, it is parabolic for the second frame and logarithmic for the third frame etc. After transmitting the fifth frame with its function of coding transmitting the next frame occurs with the function of coding corresponding to the first frame of the cycle, e.g. the function of coding becomes linear again.

The embodiment of the method can be illustrated with the following example shown in Table 1. A cycle of changing a function of coding is defined with a table. The table shows a uniform dependence between the transformed illumination of a fragment and the transformed intensity of electrical skin stimulation corresponding to the fragment of an area of the skin sensitive information zone. The transformed illumination equal to 0 corresponds to a black e.g. not illuminated fragment of the image. The illumination equal to 1 corresponds to as much as possibly bright fragment of the image. The transformed illumination of electrical skin stimulation equal to 0 corresponds to the current impulse transmitted to the area of the skin sensitive information zone with the strength of 0,6 mA and equal to 1-3,2 mA. In this case the current intensity changes within the given limits according to the linear law.

**Table 1**

| The fragment number | Transformed illumination of a fragment | | | | | |
|---|---|---|---|---|---|---|
| | 0 | 0,2 | 0,4 | 0,6 | 0,8 | 1 |
| 1 | 0 | 0,2 | 0,4 | 0,6 | 0,8 | 1 |
| 2 | 0 | 0,1 | 0,3 | 0,7 | 0,9 | 1 |
| 3 | 0 | 0,1 | 0,25 | 0,50 | 0,75 | 1 |
| 4 | 0 | 0,06 | 0,20 | 0,75 | 0,8 | 1 |
| 5 | 0 | 0,04 | 0,15 | 0,45 | 0,7 | 1 |
| | Transformed intensity of electrical skin stimulation | | | | | |

Coding the illumination of each fragment during translation of each frame is determined by the function of coding corresponding to this fragment. Thus for the first frame of the cycle the function of coding is linear and is determined by the first line of the table, for the second frame the function is determined by the second line etc. The table shows as example of a direct, or positive, function of coding, when the greater intensity of electrical skin stimulation corresponds to the greater illumination. A cycle can also consist of positively and negatively coded frames. In case of the negative coding of the frame the function of coding is inverse, i.e. the more the illumination of the fragment is the less is the illumination intensity of the corresponding to it area of the skin sensitive information zone. It results in coding the dark and the light fragments with different frequencies of electrical skin stimulation in the image being conveyed. Thus if a cycle consists of three positive and one negative frame the frequency of electrical stimulation of the light fragments is three times more than the frequency of electrical stimulation of the dark fragments. In this case the dark differs from the light not only by the intensity of electrical skin influence but it is also exceptional in the frequency of stimulation and electrical tactile sensitivity is rather high in reference to it. Transmitting signals to the skin sensitive information zone with the frequency equal to 25-120 frames a second permits to form cycles of changing function of coding consisting of 3-10 frames. In this case the information being conveyed is perceived by a person integrally and with the great resolution in comparison with well-known method.

The function of coding not only includes determining dependence between the illumination of a fragment and the intensity of stimulation. Electrical skin stimulation of the areas of the skin sensitive information zone is carried out with current impulses than can follow with different frequencies and have different duration. Therefore the cyclicity of changing the function of coding may relate both to the change of the dependence of current intensity and/or voltage from the illumination and to the change frequency of the current impulses proceeding and/or of their duration. In this case the method can be carried out as follows. The dependence of the impulse current intensity from the illumination is the same for all the frames; i.e. it may be linear. However the frequency of current impulses proceeding differs for each frame of the cycle. For example, a cycle consists of 4 frames, then the frequency of current impulses proceeding is equal to 150 Hz for the first frame of the cycle of changing the function of coding, for the second frame the frequency is equal to 180 Hz. It is equal to 200 Hz for the third frame and 220 Hz for the fourth one.

In particular the frames coded positively include signals as current impulses with frequency for example of 100 Hz and the frames coded negatively consist of current impulses with frequencies for example 150 Hz. In this case the opportunity for additive distinguishing the information coming to the skin sensitive zone are formed.

Realisation of this method when the difference in the fragments' illumination is coded with the difference in the intensity of electrical skin stimulation of the areas of the skin sensitive information zone or of electrical tactile stimulation but the signals about the image are being transmitted to the information zone of the skin not discretely (frame by frame) but with analogous way (continuously). In this case the cycle of changing the function of coding may consist for example of 5 time intervals. In this case for the first time interval the function of coding may be linear, during the second interval the function may be parabolic, the third function may be logarithmic etc. Coding illumination of every fragment during transmitting an image is determined with the function of coding corresponding to the current time interval of the cycle of the function of coding. After transmitting the signals of the fifth time interval with its function of coding transmitting the signals following after the previous ones is to be carried out with the function of coding corresponding to the first time interval of the cycle, i.e. the function of coding becomes linear again. In this case a cycle may exist of time intervals with positively and negatively coded distinctions in the illumination of an image. In case of the negative coding of the time interval of a cycle the function of coding is inverse, i.e. the greater the illumination of a fragment is the less is the intensity of stimulation of the corresponding to it area of the skin sensitive information zone. It leads to coding dark and light fragments of the image being transmitted with different fragment of electrical skin stimulation. So if a cycle consists of 3 positive and one negative time intervals the frequency of electrical stimulation of the light fragments is three times more than the frequency of electrical stimulation of the dark fragments. In this case the dark differs from the light not only in intensity of electrical skin influence but it also stands out for the frequency of stimulation.

### APPLIANCE OF THE INVENTION

This method was tested by experiments repeatedly and thoroughly. The experiments confirmed its efficiency and advantages in comparison with the known methods of the analogous purpose.

## Claims

1. The method of conveying to the nervous system of a person including disintegration of the initial information into separate streams, coding each stream as a sequence of signals and transmitting the signals of each stream to separate fixed areas of the tactile sensitivity of a person in total forming information zone characterised in that the process of conveying the information the function of coding is being cyclically changed ensuring integration of perceiving information by a person within the limits of the cycle by choosing the parameters of the function and the law of changing the function in time.

2. The method of conveying information zone according to claim 1 characterised in that conveying the visual information is to be carried out discretely frame by frame with cyclical as for frames changing the dependence of intensity of a signal and/or its frequency from the brightness of the corresponding fragments of the frames and the integration of perceiving the frames within the limits of the cycle is ensured with choosing time characteristics of the cycles.

3. The method of conveying information according to claim 2, characterised in that according to point 2 the dependence of intensity and/or frequency of a signal from the brightness of the corresponding fragment is direct for some frames of the cycle (positive frames) and is inverse (negative frames) for the other part of the frames of the cycle.

4. The method of conveying information according to claim 3, characterised in that the number of the positive frames is more than the number of the negative frames within the limits of the cycle.

5. The method of conveying information according to claims 3 or 4, characterised in that the number of positive frames is less than the number of the negative frames within the limits of the cycle.

6. The method of conveying information according to claim 3 or 4, characterised in that the frequency of signals in negative and positive frames is different.

7. A device for implementing the method of one of claims 1 to 6,
comprising a first means for transforming initial information, preferably visual or audible information into signal sensible preferably tactily by a human being when applied to its integument, the transformation being performed according to a transformation function, characterised in that
a second means is present for cyclically modifying the transformation function and/or the sensible signal in order to avoid desensibilisation of the senses of the being's integument.

8. A device according to claim 7 characterised in that the sensible signal comprises an electric signal and consists of pulses whose duration is modifiable by the second means, and/or the sensible signal comprises an a.c. carrier, preferably in the frequency range from 150 Hz to 220 Hz, the frequency of the carrier being modifiable by the second means,
wherein one or more of current, voltage, or frequency of the electric signal is controlled by the transformation.

9. A device according to claim 7 or 8, characterised in that the first means comprises a vibrating means, the vibration of which constitutes at least part of the sensible signal, wherein the frequency and/or amplitude of the vibration are controlled by the transformation of the initial information.

10. The device according to one of claims 7 to 9, characterised in that the second means provides a set of transformation functions being cyclically furnished to the first means for being used as the actual transformation function, and the set of transformation function comprising preferably:
- a proportional function,
- a polynomial function,
- a logarithm function,
- a parabolic function,
- an inversion or
- a function combined thereof.
